# EUROPEAN PATENT SPECIFICATION

(11) **EP 2 971 028 B1**
(45) Date of publication and mention of the grant of the patent: **22.04.2020**
(21) Application number: 14713012.4
(22) Date of filing: 11.03.2014
(51) Int. Cl.: C12P 7/10, C13K 1/02, C13K 13/00, D21C 1/00, C12P 19/02, C12P 19/14, D21C 3/02, C08H 8/00

(54) **GRADIENT PRETREATMENT OF LIGNOCELLULOSIC BIOMASS**
GRADIENTENVORBEHANDLUNG VON LIGNOCELLULOSISCHER BIOMASSE
PRÉTRAITEMENT EN GRADIENT D'UNE BIOMASSE LIGNOCELLULOSIQUE

(30) Priority: 12.03.2013 US 201361776946 P
(43) Date of publication of application: 20.01.2016
(73) Proprietor: E. I. du Pont de Nemours and Company, Wilmington, DE 19805 (US)
(72) Inventor: CHEUNG, Patricia, Glen Mills, Pennsylvania 19342 (US); FOX, Bradley, Curt, Bear, Delaware 19701 (US); LAU, Ming, Woei, Maryville, Tennessee 37804 (US); SELBY, Joseph, Michael, Knoxville, Tennessee 37922 (US); SHANKWITZ, Gregory, Paul, Landenberg, Pennsylvania 19350 (US); THOMAS, Stuart, M., Wilmington, Delaware 19809 (US); WARNER, Ryan, Eric, Maryville, Tennessee 37804 (US)
(74) Representative: Dehns
(86) International application number: PCT/US2014/022902
(87) International publication number: WO 2014/164581

(56) References cited:
- EP-A1- 2 336 291
- WO-A2-2012/071312
- WO-A2-2012/088429
- DIEN B S ET AL: "Enzyme characterization for hydrolysis of AFEX and liquid hot-water pretreated distillers' grains and their conversion to ethanol", BIORESOURCE TECHNOLOGY, ELSEVIER BV, GB, vol. 99, no. 12, 1 August 2008 (2008-08-01), pages 5216-5225, XP022654841, ISSN: 0960-8524, DOI: 10.1016/J.BIORTECH.2007.09.030 [retrieved on 2007-11-08]
- LAU MING W ET AL: "Ethanolic fermentation of hydrolysates from ammonia fiber expansion (AFEX) treated corn stover and distillers grain without detoxification and external nutrient supplementation", BIOTECHNOLOGY AND BIOENGINEERING, WILEY & SONS, HOBOKEN, NJ, US, vol. 99, no. 3, 1 February 2008 (2008-02-01), pages 529-539, XP002522477, ISSN: 0006-3592, DOI: 10.1002/BIT.21609 [retrieved on 2007-08-17]
- KAZI F K ET AL: "Techno-economic comparison of process technologies for biochemical ethanol production from corn stover", FUEL, IPC SCIENCE AND TECHNOLOGY PRESS, GUILDFORD, GB, vol. 89, 1 November 2010 (2010-11-01), pages S20-S28, XP027409970, ISSN: 0016-2361 [retrieved on 2010-01-09]

## Description

### FIELD OF THE INVENTION

Processes for treating biomass to obtain fermentable sugars are provided. Specifically, the invention relates to a pretreatment process for lignocellulosic biomass using ammonia where the percent of biomass solids is reduced over time.

### BACKGROUND OF THE INVENTION

Lignocellulosic feedstocks and wastes, such as agricultural residues, wood, forestry wastes, sludge from paper manufacture, and municipal and industrial solid wastes, provide a potentially large renewable feedstock for the production of chemicals, plastics, fuels and feeds. Lignocellulosic feedstocks and wastes containing the carbohydrate polymers cellulose and hemicellulose, as well as lignin, are generally treated by a variety of chemical, mechanical and enzymatic means to release primarily hexose and pentose sugars, which can then be fermented to useful products.

Pretreatment methods are used to make the carbohydrate polymers, or polysaccharides, of lignocellulosic biomass more readily accessible to cellulolytic enzymes used in saccharification. Various pretreatment methods are known, including ammonia pretreatment of biomass. For example, US 7,932,063 discloses methods for pretreating biomass under conditions of high solids and low aqueous ammonia concentration. The concentration of ammonia used is minimally a concentration that is sufficient to maintain the pH of the biomass-aqueous ammonia mixture alkaline and maximally less than about 12 weight percent relative to dry weight of biomass. The dry weight of biomass is at least about 15% up to about 80% of the weight of the biomass-aqueous ammonia mixture.

WO 2012/088429 discloses a biomass treatment process in which gaseous ammonia is allowed to condense on the biomass and react with the water in it, whereby the reactivity of polysaccharides in the biomass is said to be increased during subsequent biological conversion.

US 2010/0184176 discloses a biomass hydrothermal decomposition apparatus in which biomass is fed into the main body and hot compressed water is fed from another end such that a gradient of biomass concentration occurs within the apparatus.

There remains a need for a process for pretreating lignocellulosic biomass with ammonia, producing a readily saccharifiable material, that supports enhanced production of fermentable sugars and of a biocatalyst-produced product during fermentation.

### SUMMARY OF THE INVENTION

The invention provides a process for pretreating lignocellulosic biomass that supports enhanced production of a biocatalyst-produced product following saccharification.

Accordingly, the invention provides a process for producing fermentable sugars from cellulosic biomass comprising:
a) providing in a reaction vessel a reaction mixture comprising:
   i) an initial solids concentration of cellulosic biomass of between 40% and 70%, measured as percent of dry biomass relative to the total mixture on a weight to weight basis; and
   ii) ammonia, having a loading concentration of between 4% and 24% relative to dry weight of the biomass;
b) maintaining the reaction mixture between a temperature of 100 °C and 230 °C for a pretreatment reaction time having a starting time of t₀ and a final time of tₙ to produce a pretreated biomass, wherein at time t₀ the solids concentration of the reaction mixture is the initial solids concentration; and
c) contacting the pretreated biomass with a saccharification enzyme consortium under suitable conditions to produce fermentable sugars;
characterised by increasing the liquid content of the reaction mixture over the pretreatment reaction time of t₀ to tₙ in a gradient manner, whereby the solids concentration decreases by between 20% and 30% as compared to the initial solids concentration, to produce the pretreated biomass.

### BRIEF DESCRIPTION OF THE FIGURES

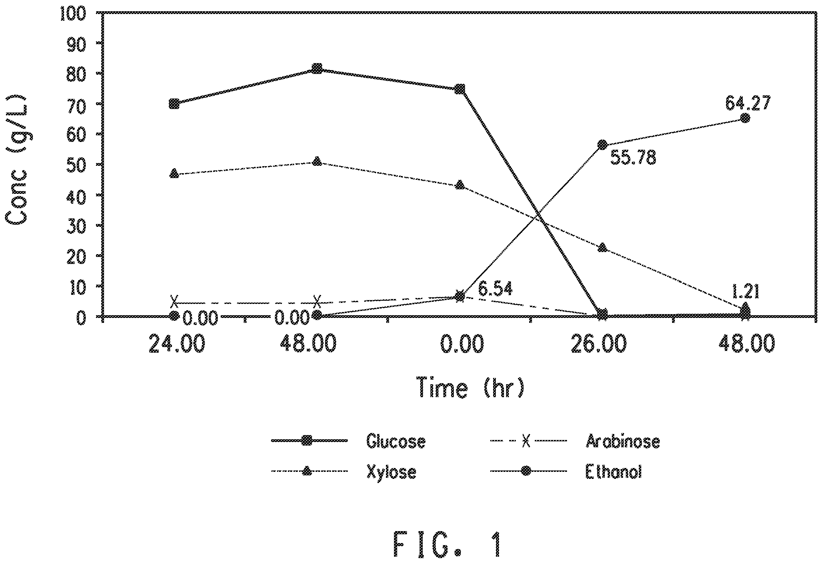

Figure 1 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass at 40% solids performed in one stage.

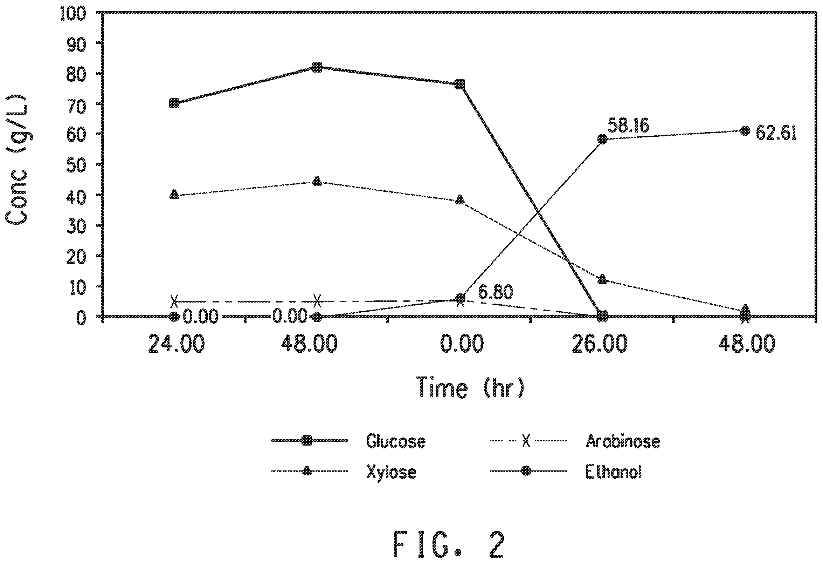

Figure 2 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass at 52.5% solids performed in one stage.

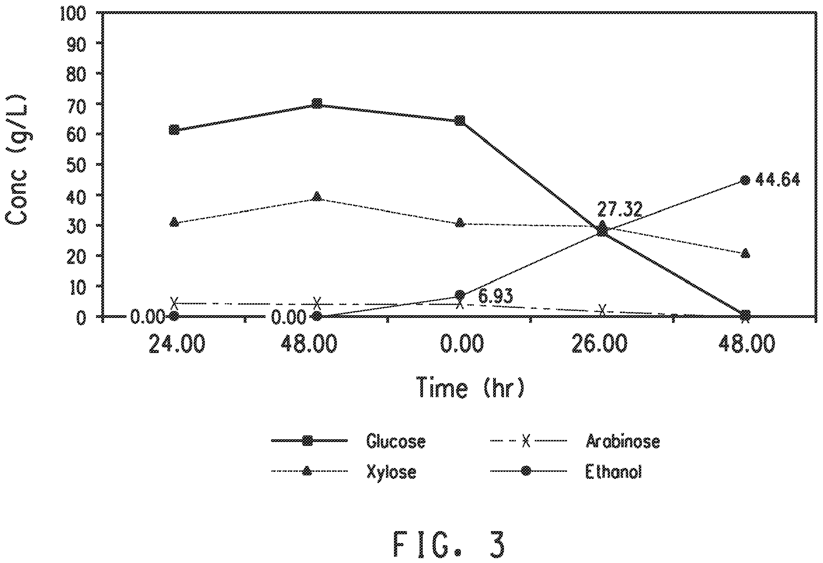

Figure 3 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass at 65% solids performed in one stage.

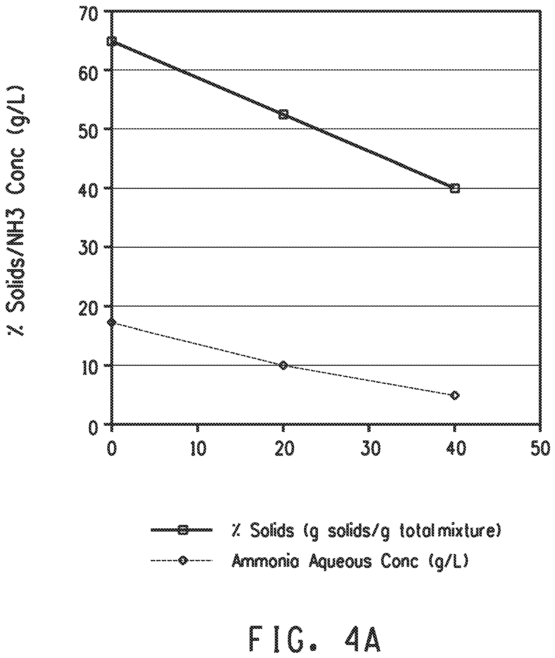

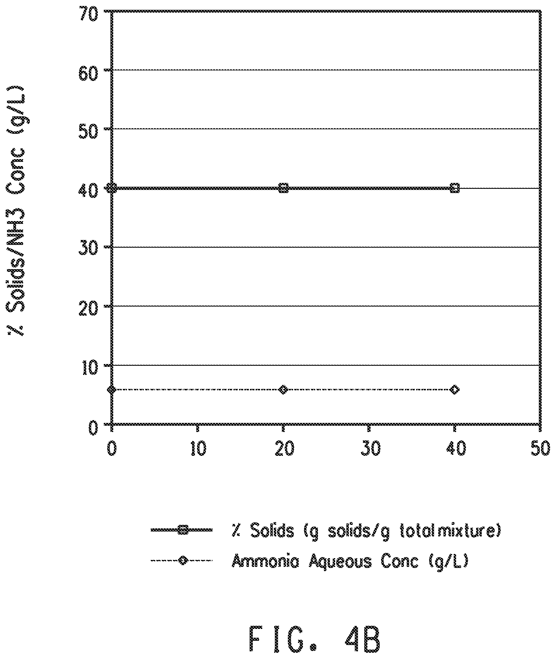

Figure 4 shows graphs of % solids and ammonia concentration over time in a pretreatment with a gradient of %solids (A), and in a pretreatment with constant solids at 40% (B).

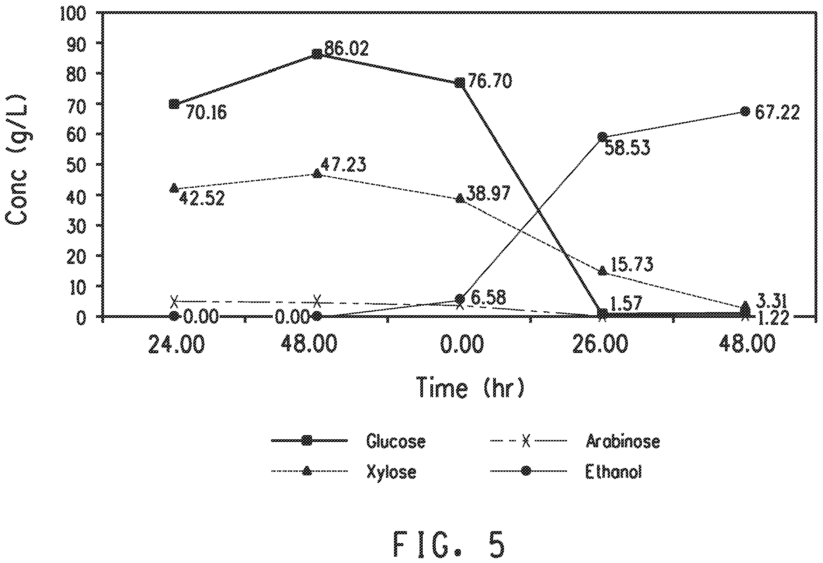

Figure 5 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass and a gradient of 65%, then 52.5%, then 40% solids performed in three stages.

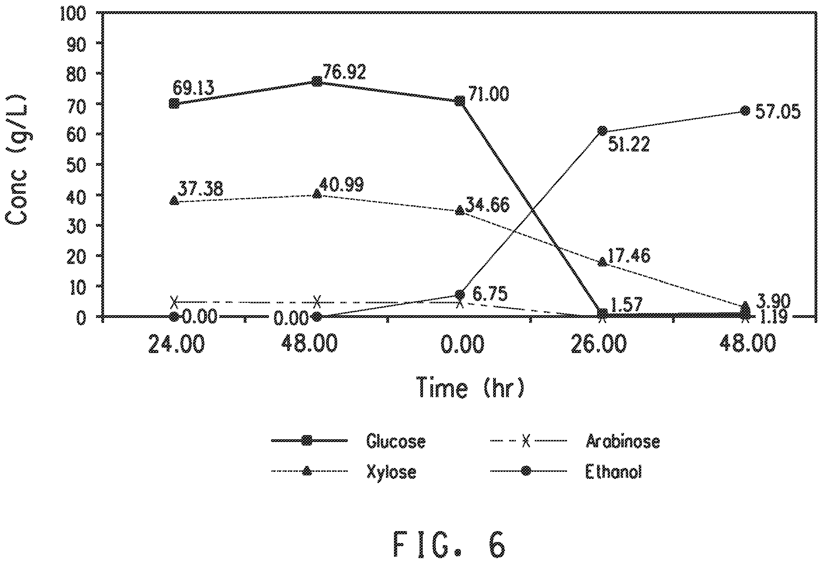

Figure 6 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass at 65% solids performed in three stages.

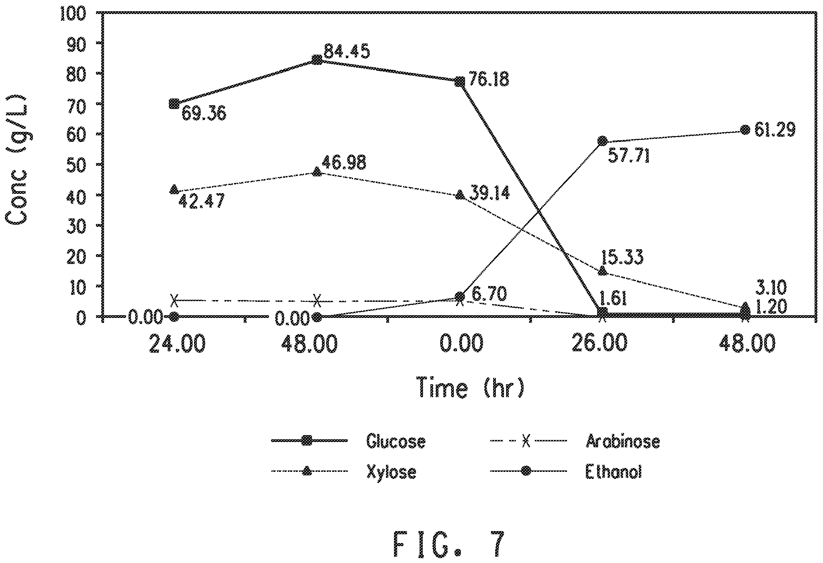

Figure 7 is a graph showing glucose, xylose, and arabinose concentrations during sacharification (at 24, 48 hr), and glucose, xylose, arabinose, and ethanol concentrations during fermentation (at 0, 26, and second 48 hr) starting with stover pretreated with 8% NH₃/g dry biomass at 40% solids performed in three stages.

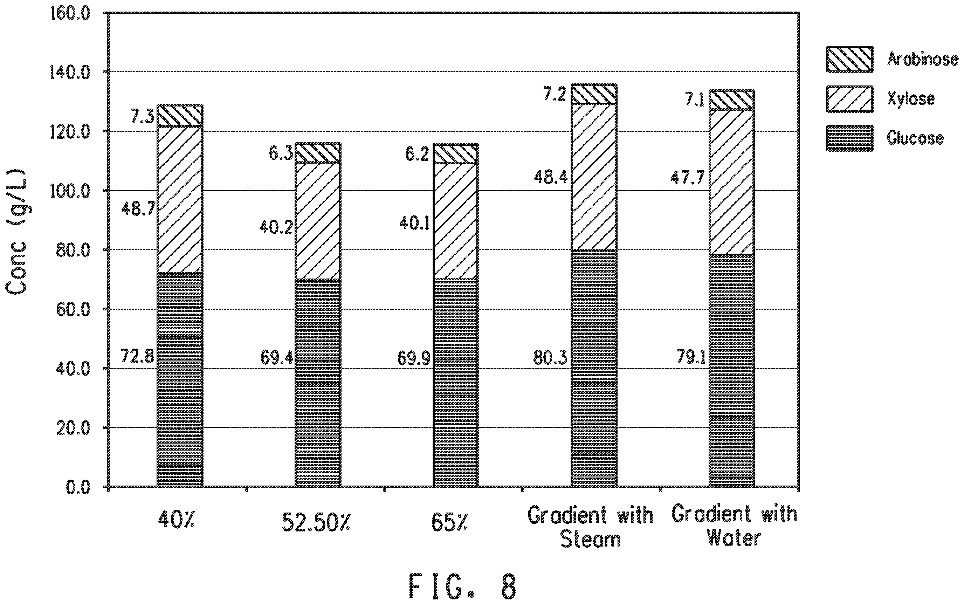

Figure 8 is a graph showing glucose, xylose, and arabinose yields after 48 hr of saccharification of corn stover pretreated using constant 40%, 52.5%, or 65% solids, or gradient 65% to 40% solids with the gradient produced using either steam injection or dry heat and water addition.

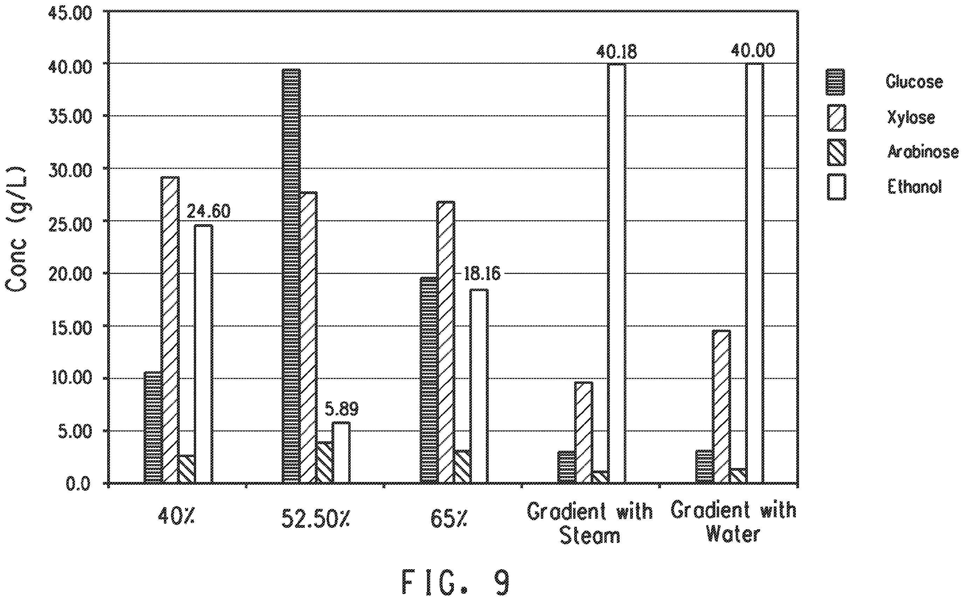

Figure 9 is a graph showing glucose, xylose, arabinose and ethanol concentrations after 120 hr of fermentation using hydrolysate produced from corn stover pretreated using constant 40%, 52.5%, or 65% solids, or gradient 65% to 40% solids with the gradient produced using either steam injection or dry heat and water addition, where fermentation was started at 2% v/v inoculum.

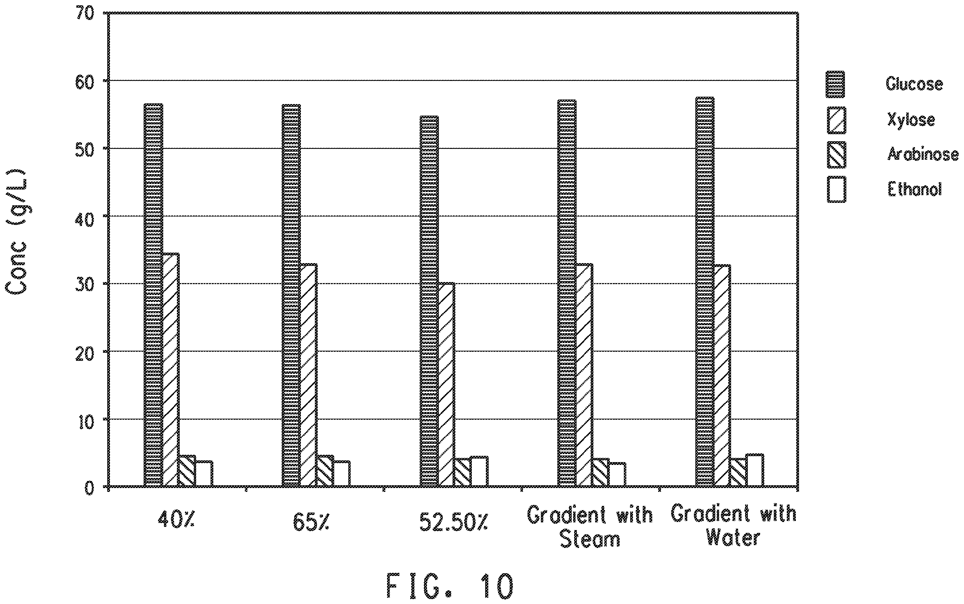

Figure 10 is a graph showing glucose, xylose, arabinose and ethanol concentrations at time = 0 for fermentation using hydrolysate produced from corn stover pretreated using constant 40%, 52.5%, or 65% solids, or gradient 65% to 40% solids with the gradient produced using either steam injection or dry heat and water addition, where fermentation was started at 10% v/v inoculum.

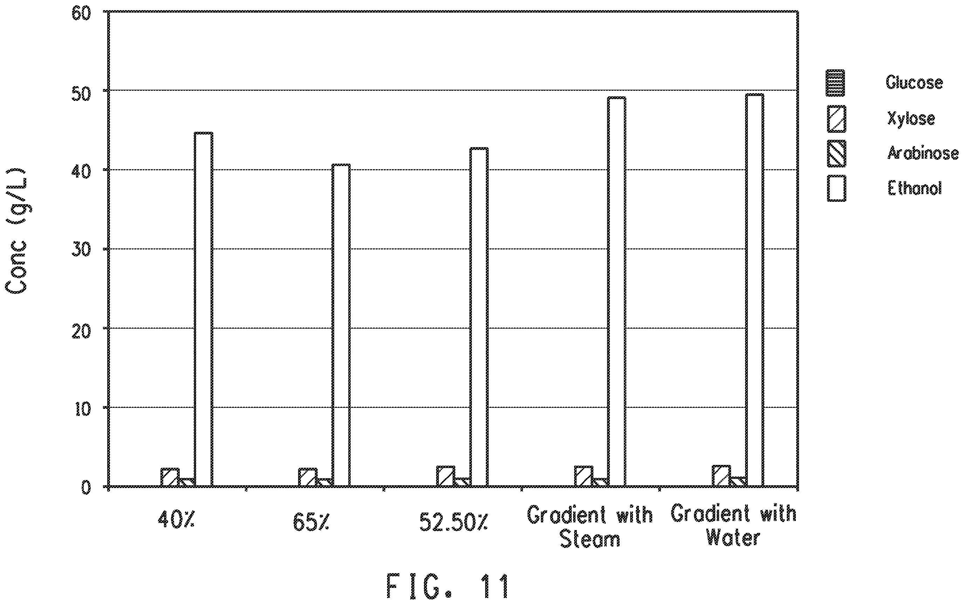

Figure 11 is a graph showing glucose, xylose, arabinose and ethanol concentrations after 48 hr of fermentation using hydrolysate produced from corn stover pretreated using constant 40%, 52.5%, or 65% solids, or gradient 65% to 40% solids with the gradient produced using either steam injection or dry heat and water addition, where fermentation was started at 10% v/v inoculum.

### DETAILED DESCRIPTION

The invention relates to a process for pretreating lignocellulosic biomass and saccharifying the pretreated biomass to produce fermentable sugars. There are opposing factors related to the optimal concentration of biomass solids used during pretreatment, including the amount of sugars that can be released from the biomass and the amount of inhibitors that are also released, as well as the accessability of the biomass to pretreatment agents. Under conditions described herein, it is effective to start with a high biomass solids concentration and reduce the biomass solids concentration during a pretreatment reaction. Using this method, the pretreated biomass product is more effectively saccharified, producing higher yields of fermentable sugars leading to enhanced biocatalyst product synthesis during fermentation.

The following definitions and abbreviations are to be used for the interpretation of the claims and the specification.

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "invention" or "present invention" as used herein is a nonlimiting term and is not intended to refer to any single embodiment of the particular invention but encompasses all possible embodiments as described in the specification and the claims.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities. In one embodiment, the term "about" means within 10% of the reported numerical value, preferably within 5% of the reported numerical value.

The term "fermentable sugar" refers to oligosaccharides and monosaccharides that can be used as a carbon source by a microorganism in a fermentation process.

The term "lignocellulosic" refers to a composition comprising both lignin and cellulose. Lignocellulosic material may also comprise hemicellulose.

The term "cellulosic" refers to a composition comprising cellulose and additional components, including hemicellulose.

The term "saccharification" refers to the production of fermentable sugars from polysaccharides.

The term "pretreated biomass" means biomass that has been subjected to pretreatment prior to saccharification.

The term "butanol" refers to isobutanol, 1-butanol, 2-butanol, or combinations thereof.

The term "lignocellulosic biomass" refers to any lignocellulosic material and includes materials comprising cellulose, hemicellulose, lignin, starch, oligosaccharides and/or monosaccharides. Biomass may also comprise additional components, such as protein, lipid, ash, and/or extractives. Biomass may be derived from a single source, or biomass can comprise a mixture derived from more than one source; for example, biomass could comprise a mixture of corn cobs and corn stover, or a mixture of grass and leaves. Lignocellulosic biomass includes, but is not limited to, bioenergy crops, agricultural residues, municipal solid waste, industrial solid waste, sludge from paper manufacture, yard waste, wood and forestry waste. Examples of biomass include, but are not limited to, corn cobs, crop residues such as corn husks, corn stover, corn grain fiber, grasses, beet pulp, wheat straw, wheat chaff, oat straw, barley straw, barley hulls, hay, rice straw, rice hulls, switchgrass, miscanthus, cord grass, reed canary grass, waste paper, sugar cane bagasse, sorghum bagasse, sorghum stover, soybean stover, components obtained from milling of grains, trees, branches, roots, leaves, wood chips, sawdust, palm waste, shrubs and bushes, vegetables, fruits, flowers, and animal manure.

The term "dry matter content" refers to the amount by weight of the subject material that exists after liquid content of the material is removed.

The term "biomass hydrolysate" refers to the product resulting from saccharification of biomass. The biomass may also be pretreated or preprocessed prior to saccharification.

The term "biomass hydrolysate fermentation broth" is broth containing product resulting from biocatalyst growth and production in a medium comprising biomass hydrolysate. This broth includes components of biomass hydrolysate that are not consumed by the biocatalyst, as well as the biocatalyst itself and product made by the biocatalyst.

The term "target compound" or "target product" refers to any product that is produced by a microbial production host cell in a fermentation. Target compounds may be the result of genetically engineered enzymatic pathways in host cells or may be produced by endogenous pathways. Typical target compounds include but are not limited to acids, alcohols, alkanes, alkenes, aromatics, aldehydes, ketones, biopolymers, proteins, peptides, amino acids, vitamins, antibiotics, and pharmaceuticals.

### Pretreatment using biomass solids gradient

In the present method, the concentration of biomass solids in a pretreatment reaction is reduced over the time course of the reaction. The concentration of biomass is a solids concentration, which is the percent of dry biomass relative to the total pretreatment reaction mixture on a weight to weight basis.

Biomass refers to any cellulosic or lignocellulosic material, for example, bioenergy crops, agricultural residues, municipal solid waste, industrial solid waste, yard waste, wood, forestry waste and combinations thereof. In various embodiments the biomass is first size reduced, such as by grinding, milling, shredding, chopping, disc refining, and/or cutting. In addition, the biomass may be dried, such as air dried or dried with heat.

The pretreatment reaction mixture includes biomass and ammonia as a pretreatment agent. The ammonia may be used in a liquid, or anhydrous state. The ammonia may be ammonia solution, anhydrous ammonia, ammonia vapor, or any mixtures of these. In one embodiment a portion or all of the ammonia may be recycled. The ammonia loading in the pretreatment reaction mixture is in an amount that is between 4% and 24% relative to the dry weight of the biomass in the reaction mixture. In one embodiment the ammonia is between 4% and about 12 wt.% relative to dry weight of biomass. The amount of ammonia relative to the dry weight of biomass may vary during the pretreatment reaction, remaining between 4% and 24%. In one embodiment the concentration of ammonia in the reaction mixture decreases as the liquid content of the reaction mixture increases. In another embodiment, additional ammonia is added to maintain the ammonia concentration as the liquid content increases.

The solids concentration of biomass at the starting time t₀ for the pretreatment reaction is between 40% and 70%. In various embodiments the solids concentration is 40%, 45%, 50%, 55%, 60%, 65%, or 70%. The biomass is mixed with ammonia, and water if needed, to reach the desired concentration, either prior to or after loading into a reaction vessel. The liquid content of the vessel is increased over time to reduce the biomass solids concentration such that the concentration is reduced by between 20% and 30% at the end of pretreatment, which is final time tₙ, as compared to the initial solids concentration. In one embodiment the liquid concentration is increased by adding steam to the reaction vessel. In another embodiment the liquid concentration is increased by adding water to the reaction vessel. In other embodiments, the liquid concentration is increased by adding ammonia solution to the reaction vessel, either alone or in combination with steam or water. Added ammonia may be in an amount that maintains a constant concentration with respect to the biomass solids concentration. Alternatively, ammonia may be added to keep the concentration constant in the reaction mixture, or to have a varying concentration during the reaction that is within the described range.

In one embodiment the decrease in solids concentration from t₀ to tₙ is linear. In another embodiment the decrease in solids concentration is monotonic but not linear. Typically the rate of decrease is fast at the beginning of the reaction, and slows towards time tₙ. In another embodiment the decrease in solids concentration is step wise, with the solids concentration being held constant one or more times between t₀ and tₙ.

The reaction vessel is heated such that the temperature of the reaction mixture during pretreatment is maintained between 100 °C and 230 °C. In various embodiments the reaction mixture has a temperature of 100, 120, 140, 160, 180, or 200 °C, or may vary among a number of these temperatures, during pretreatment. In additional embodiments the temperature is maintained between 100 °C and about 220 °C, or between 100 °C and about 200 °C, and may vary among a number of these temperatures, during pretreatment. Variations may be either increases or decreases in temperature. The reaction vessel may be heated by any means, such as externally, or internally by adding steam.

The temperature of the pretreatment reaction mixture is maintained between 100 °C and about 200 °C, or between 100 °C and about 220 °C, or between 100 °C and 230 °C for a reaction time that is between about 10 minutes and 2 hours for production of a pretreated biomass. Typically the reaction time is between about 20 minutes and 60 minutes. Additionally the reaction time may be 1, 2, 3, 4, 5, 6, 7, 8, or 9 minutes. Generally, shorter reactions times are effective at higher reaction temperatures.

The pretreatment of biomass is carried out in any suitable reaction vessel. Typically the vessel is one that can withstand pressure, can be heated or has a mechanism for heating, and has a mechanism for mixing the contents. A vessel that lacks mixing, or with mixing not activated, may also be used. Commercially available vessels include, for example, a Parr reactor (Parr Instrument, Moline, IL),a Zipperclave® reactor (Autoclave Engineers, Erie, PA), a Jaygo reactor (Jaygo Manufacturing, Inc., Mahwah, NJ), and a steam gun reactor (Autoclave Engineers, Erie, PA). Much larger scale reactors with similar capabilities may be used.

Biomass pretreated by the present gradient biomass solids concentration process was shown herein in Example 3 to produce fermentable sugars, following saccharification as described below, at greater yields than from biomass pretreated in comparative constant biomass solids concentration processes. The total sugar yields were at least about 4% greater for the gradient pretreatment samples than for the constant pretreatment samples. In various embodiments the sugars yield is at least about 3%, 4%, 5%, 6%, 7%, 8%, 9%, 10% or more, above the yield from biomass pretreated in a comparable but non-gradient process.

Fermentation of the fermentable sugars in hydrolysates from biomass pretreated by the present gradient biomass solids concentration process, using a *Zymomonas* biocatalyst that produces ethanol, was shown herein in Example 3 to be more effective than fermentation of hydrolysates from biomass pretreated in comparative constant biomass solids concentration processes, based on glucose and xylose utilization, and ethanol production.

### Saccharification

In the present method the pretreated biomass is contacted with a saccharification enzyme consortium under suitable conditions to produce fermentable sugars, and the fermentable sugars provide a carbohydrate source for a biocatalyst for a fermentation process. Prior to saccharification, the pretreated biomass may be treated to alter the pH, composition or temperature such that the enzymes of the saccharification enzyme consortium will be active. The pH may be altered through the addition of acids in solid or liquid form. Alternatively, carbon dioxide (CO₂), which may be recovered from fermentation, may be utilized to lower the pH. For example, CO₂ may be collected from a fermenter and fed into the pretreatment product headspace in the flash tank or bubbled through the pretreated biomass if adequate liquid is present while monitoring the pH, until the desired pH is achieved. The temperature is brought to a temperature that is compatible with saccharification enzyme activity, as noted below. Typically suitable conditions may include temperature between about 40 °C and 50 °C and pH between about 4.8 and 5.8.

Enzymatic saccharification of cellulosic or lignocellulosic biomass typically makes use of an enzyme composition or blend to break down cellulose and/or hemicellulose and to produce a hydrolysate containing sugars such as, for example, glucose, xylose, and arabinose. Saccharification enzymes are reviewed in Lynd, L. R., et al. (Microbiol. Mol. Biol. Rev., 66:506-577, 2002). At least one enzyme is used, and typically a saccharification enzyme blend is used that includes one or more glycosidases. Glycosidases hydrolyze the ether linkages of di-, oligo- and polysaccharides and are found in the enzyme classification EC 3.2.1.x (Enzyme Nomenclature 1992, Academic Press, San Diego, CA with Supplement 1 (1993), Supplement 2 (1994), Supplement 3 (1995, Supplement 4 (1997) and Supplement 5 [in Eur. J. Biochem., 223:1-5, 1994; Eur. J. Biochem., 232:1-6, 1995; Eur. J. Biochem., 237:1-5, 1996; Eur. J. Biochem., 250:1-6, 1997; and Eur. J. Biochem., 264:610-650 1999, respectively]) of the general group "hydrolases" (EC 3.). Glycosidases useful in the present method can be categorized by the biomass components they hydrolyze. Glycosidases useful for the present method may include cellulose-hydrolyzing glycosidases (for example, cellulases, endoglucanases, exoglucanases, cellobiohydrolases, β-glucosidases), hemicellulose-hydrolyzing glycosidases (for example, xylanases, endoxylanases, exoxylanases, β-xylosidases, arabino-xylanases, mannases, galactases, pectinases, glucuronidases), and starch-hydrolyzing glycosidases (for example, amylases, α-amylases, β-amylases, glucoamylases, α-glucosidases, isoamylases). In addition, it may be useful to add other activities to the saccharification enzyme consortium such as peptidases (EC 3.4.x.y), lipases (EC 3.1.1.x and 3.1.4.x), ligninases (EC 1.11.1.x), or feruloyl esterases (EC 3.1.1.73) to promote the release of polysaccharides from other components of the biomass. It is known in the art that microorganisms that produce polysaccharide-hydrolyzing enzymes often exhibit an activity, such as a capacity to degrade cellulose, which is catalyzed by several enzymes or a group of enzymes having different substrate specificities. Thus, a "cellulase" from a microorganism may comprise a group of enzymes, one or more or all of which may contribute to the cellulose-degrading activity. Commercial or non-commercial enzyme preparations, such as cellulase, may comprise numerous enzymes depending on the purification scheme utilized to obtain the enzyme. Many glycosyl hydrolase enzymes and compositions thereof that are useful for saccharification are disclosed in WO 2011/038019.

Saccharification enzymes may be obtained commercially. Such enzymes include, for example, Spezyme® CP cellulase, Multifect® xylanase, Accelerase® 1500, Accellerase® DUET, and Accellerase® Trio™ (Dupont™/ Genencor®, Wilmington, DE), and Novozyme-188 (Novozymes, 2880 Bagsvaerd, Denmark). In addition, saccharification enzymes may be unpurified and provided as a cell extract or a whole cell preparation. The enzymes may be produced using recombinant microorganisms that have been engineered to express one or more saccharifying enzymes.

Additional enzymes for saccharification include, for example, glycosyl hydrolases such as members of families GH3, GH39, GH43, GH55, GH10, and GH11. GHs are a group of enzymes that hydrolyze the glycosidic bond between two or more carbohydrates, or between a carbohydrate and a noncarbohydrate moiety. Families of GHs have been classified based on sequence similarity and the classification is available in the Carbohydrate-Active enzyme (CAZy) database (Cantarel et al. (2009) Nucleic Acids Res. 37 (Database issue):D233-238). Certain of these enzymes are able to act on various substrates and have demonstrated effecacy as saccharification enzymes. Glycoside hydrolase family 3 ("GH3") enzymes have a number of known activities, including, for example, β-glucosidase (EC:3.2.1.21); β-xylosidase (EC:3.2.1.37); N-acetyl β- glucosaminidase (EC:3.2.1.52); glucan β-1,3-glucosidase (EC:3.2.1.58); cellodextrinase (EC:3.2.1.74); exo-1,3-1,4-glucanase (EC:3.2.1); and/or β-galactosidase (EC 3.2.1.23) activities. Glycoside hydrolase family 39 ("GH39") enzymes also have a number of known activities, including, for example, α-L-iduronidase (EC:3.2.1.76) and/or β-xylosidase (EC:3.2.1.37) activities. Glycoside hydrolase family 43 ("GH43") enzymes have a number of known activities including, for example, L-α-arabinofuranosidase (EC 3.2.1.55); β-xylosidase (EC 3.2.1.37); endoarabinanase (EC 3.2.1.99); and/or galactan 1,3-β-galactosidase (EC 3.2.1.145) activities. Glycoside hydrolase family 51 ("GH51") enzymes are known to have, for example, L-α-arabinofuranosidase (EC 3.2.1.55) and/or endoglucanase (EC 3.2.1.4) activities. Glycoside hydrolase family 10 ("GH10") have beendescribed in detail in Schmidt et al., 1999, Biochemistry 38:2403-2412 and Lo Leggio et al., 2001, FEBS Lett 509: 303-308) and the Glycoside hydrolase family 11 ("GH11") have been described in Hakouvainen et al., 1996, Biochemistry 35:9617-24.

### Biocatalyst

Any biocatalyst that produces a target compound utilizing glucose and preferably also xylose, either naturally or through genetic engineering, may be used for fermentation of the fermentable sugars produced using the present process. Target compounds that may be produced by fermentation include, for example, acids, alcohols, alkanes, alkenes, aromatics, aldehydes, ketones, biopolymers, proteins, peptides, amino acids, vitamins, antibiotics, and pharmaceuticals. Alcohols include, but are not limited to methanol, ethanol, propanol, isopropanol, butanol, ethylene glycol, propanediol, butanediol, glycerol, erythritol, xylitol, mannitol, and sorbitol. Acids may include acetic acid, formic acid, lactic acid, propionic acid, 3-hydroxypropionic acid, butyric acid, gluconic acid, itaconic acid, citric acid, succinic acid, 3-hydroxyproprionic acid, fumaric acid, maleic acid, and levulinic acid. Amino acids may include glutamic acid, aspartic acid, methionine, lysine, glycine, arginine, threonine, phenylalanine and tyrosine. Additional target compounds include methane, ethylene, acetone and industrial enzymes.

The fermentation of sugars to target compounds may be carried out by one or more appropriate biocatalysts in single or multistep fermentations. Biocatalysts may be microorganisms selected from bacteria, filamentous fungi and yeast. Biocatalysts may be wild type microorganisms or recombinant microorganisms, and may include, for example, organisms belonging to the genera of *Escherichia, Zymomonas, Saccharomyces, Candida, Pichia, Streptomyces, Bacillus, Lactobacillus,* and *Clostridiuma.* Typical examples of biocatalysts include recombinant *Escherichia coli, Zymomonas mobilis, Bacillus stearothermophilus, Saccharomyces cerevisiae, Clostridia thermocellum, Thermoanaerobacterium saccharolyticum,* and *Pichia stipitis.*

Typically the biocatalyst is able to utilize glucose and xylose, and may additionally utilize arabinose. For example, any strain of *Zymomonas* that is an effective biocatalyst for the desired target compound production may be used. *Zymomonas* cells naturally produce ethanol using glucose, fructose and/or sucrose as fermentation substrates, but xylose is not metabolized. It is desirable to use *Zymomonas* cells that have been engineered for xylose utilization, which has been accomplished as follows. Typically four genes have been introduced into *Z. mobilis* for expression of four enzymes involved in xylose metabolism to create a xylose utilization metabolic pathway as described in US 5,514,583, US 5,712,133, US 6,566,107, WO 95/28476, Feldmann et al. ((1992) Appl Microbiol Biotechnol 38: 354-361), and Zhang et al. ((1995) Science 267:240-243). These include genes encoding xylose isomerase which catalyzes the conversion of xylose to xylulose, and xylulokinase which phosphorylates xylulose to form xylulose 5-phosphate. Additionally expressed are transketolase and transaldolase, two enzymes of the pentose phosphate pathway that convert xylulose 5-phosphate to intermediates that couple pentose metabolism to the glycolytic Entner-Douderoff pathway permitting the metabolism of xylose to ethanol (see Figure 1). DNA sequences encoding these enzymes may be obtained from any of numerous microorganisms that are able to metabolize xylose, such as enteric bacteria, and some yeasts and fungi. Sources for the coding regions may include *Xanthomonas, Klebsiella, Escherichia, Rhodobacter, Flavobacterium, Acetobacter, Gluconobacter, Rhizobium, Agrobacterium, Salmonella, Pseudomonads,* and *Zymomonas.* The coding regions of *E. coli* are typically used.

The encoding DNA sequences are operably linked to promoters that are expressed in *Zymomonas* cells such as the promoter of *Z. mobilis* glyceraldehyde-3-phosphate dehydrogenase (GAP promoter), and *Z. mobilis* enolase (ENO promoter). A mutant GAP promoter with increased expression as disclosed in US 7,989,206, is also useful for expression in *Zymomonas.* The coding regions may individually be expressed from promoters, or two or more coding regions may be joined in an operon with expression from the same promoter. The resulting chimeric genes may be introduced into *Zymomonas* cells and maintained on a plasmid, or integrated into the genome using, for example, homologous recombination, site-directed integration, or random integration. Examples of strains engineered to express a xylose utilization metabolic pathway include CP4(pZB5) (US 5,514,583), ATCC31821/pZB5 (US 6,566,107), 8b (US 7,223,575; Mohagheghi et al., (2004) Biotechnol. Lett. 25; 321-325), and ZW658 (ATTCC # PTA-7858). Cells of *Zymomonas* that are engineered for expression of the xylose utilization metabolic pathway generally require a period of adaptation in xylose-containing medium prior to being able to grow in medium that contains xylose as the only sugar.

*Zymomonas* cells may be additionally engineered for arabinose utilization as described in US 5,843,760. To allow arabinose utilization, genes expressed in addition to genes of the xylose utilization pathway include:1) L-arabinose isomerase to convert L-arabinose to L-ribulose, 2) L-ribulokinase to convert L-ribulose to L-ribulose-5-phosphate, and 3) L-ribulose-5-phosphate-4-epimerase to convert L-ribulose-5-phosphate to D-xylulose (US 5,843,760). As disclosed in US 2011/0143408, improved arabinose utilization may be achieved by additionally expressing an arabinose-proton symporter, such as by expressing a coding region from an araE gene.

In addition the *Zymomonas* cells may have one or more additional genetic modifications that improve the strain such as one that increases growth rate and/or cell mass, increases utilization of xylose and/or allows use of other sugars such as arabinose, increases tolerance to inhibitory compounds such as acetate, or increases production of ethanol. For example the endogenous *himA* gene, which encodes the alpha subunit of the integration host factor, may be genetically modified to reduce its expression which improves growth in medium containig acetate as described in US 7,897,396. Acetate is present in biomass hydrolysate, thus when using medium containing biomass hydrolysate, increased tolerance to this component is desired.

In another example a genetic modification may be made that reduces glucose-fructose oxidoreductase (GFOR) activity as described in US 7,741,119. Reduced expression of GFOR, as well as of the himA gene, may be by any method such as those described above for reducing aldose reductase activity.

In another example a genetic modification may be made which increases ribose-5-phosphate isomerase (RPI) activity, as disclosed in US 2012/0156746. Increased RPI expression may be accomplished by increasing expression of the endogenous RPI encoding gene, such as with a promoter that is more highly active than the native promoter, or by expressing a heterologous gene encoding any protein or polypeptide with ribose-5-phosphate isomerase activity in *Zymomonas.*

In another example the xylose isomerase that is expressed as part of the xylose utilization metabolic pathway is expressed using a mutant, highly active promoter that is disclosed in US 7,989,206 and US 7,998,722. The mutant promoters disclosed therein are promoters of the *Zymomonas mobilis* glyceraldehyde-3-phosphate dehydrogenase gene. Also the xylose isomerase may be a Group I xylose isomerase included in the class of enzymes identified by EC 5.3.1.5 as disclosed US 2011/0318801, issued as US 8,623,623. It is disclosed therein that Group I xylose isomerases, such as one expressed from a coding region isolated from *Actinoplanes missouriensis,* have higher activity in *Zymomonas* than Group 2 xylose isomerase. Group I xylose isomerases are defined therein by molecular phylogenetic bioinformatics analysis (using PHYLIP neighbor joining algorithm as implemented in PHYLIP (Phylogeny Inference Package version 3.5c; Felsenstein (1989) Cladistics 5:164-166), GroupSim analysis (Capra and Singh (2008) Bioinformatics 24: 1473-1480), and a Profile Hidden Markov Model (using the hmmsearch algorithm of the HMMER software package; Janelia Farm Research Campus, Ashburn, VA).

In another example the *Zymomonas* cells may be adapted for growth in a stress culture containing ethanol and ammonium acetate as disclosed in US 8,247,208. These *Zymomonas* strains with improved acetate tolerance are particularly useful when using cellulosic biomass hydrolysate containing fermentation medium, which contains acetate.

Strains disclosed in the above references and strains described herein provide examples of strains that may be used as biocatalysts and include ATCC31821/pZB5, ZW658 (ATCC #PTA-7858), ZW800, ZW801-4, ZW801-4::□ ΔhimA, AcR#3, ZW705, AR3 7-321, ZW1-XA111, and R70B1.

Additional biocatalysts that produce ethanol such as yeasts and genetically modified strains of *E. coli* (Underwood et al., (2002) Appl. Environ. Microbiol.68:6263-6272), as well as biocatalysts that produce other target compounds such as those listed above may be used in fermentation of fermentable sugars produced using the present process. For example, yeast cells that are engineered to express a pathway for synthesis of butanol and *E. coli* engineered for production of 1,3-propanediol have been described. Engineering of pathways for butanol synthesis (including isobutanol, 1-butanol, and 2-butanol) in biocatalysts have been disclosed, for example in US 8,206,970, US 20070292927, US 20090155870, US 7,851,188, and US 20080182308. Engineering of pathways in biocatalysts for 1,3-propanediol have been disclosed in US 6,514,733, US 5,686,276, US 7,005,291, US 6,013,494, and US 7,629,151.

For utilization of xylose as a carbon source, a yeast cell may be engineered for expression of a complete xylose utilization pathway. Engineering of yeast such as *S. cerevisiae* for production of ethanol from xylose is described in Matsushika et al. (Appl. Microbiol. Biotechnol. (2009) 84:37-53) and in Kuyper et al. (FEMS Yeast Res. (2005) 5:399-409).

Lactic acid has been produced in fermentations by recombinant strains of *E. coli* (Zhou et al., (2003) Appl. Environ. Microbiol. 69:399-407), natural strains of *Bacillus* (US 7,098,009), and Rhizopus oryzae (Tay and Yang (2002) Biotechnol. Bioeng. 80:1-12). Recombinant strains of *E. coli* have been used as biocatalysts in fermentation to produce 1,3 propanediol (US 6,013,494, US 6,514,733), and adipic acid (Niu et al., (2002) Biotechnol. Prog. 18:201-211). Acetic acid has been made by fermentation using recombinant *Clostridia* (Cheryan et al., (1997) Adv. Appl. Microbiol. 43:1-33), and newly identified yeast strains (Freer (2002) World J. Microbiol. Biotechnol. 18:271-275). Production of succinic acid by recombinant *E. coli* and other bacteria is disclosed in US 6,159,738, and by mutant recombinant *E. coli* in Lin et al., (2005) Metab. Eng. 7:116-127). Pyruvic acid has been produced by mutant *Torulopsis glabrata* yeast (Li et al., (2001) Appl. Microbiol. Technol. 55:680-685) and by mutant *E. coli* (Yokota et al., (1994) Biosci. Biotech. Biochem. 58:2164-2167). Recombinant strains of *E. coli* have been used as biocatalysts for production of para-hydroxycinnamic acid (US20030170834) and quinic acid (US 7,642,083).

A mutant of *Propionibacterium acidipropionici* has been used in fermentation to produce propionic acid (Suwannakham and Yang (2005) Biotechnol. Bioeng. 91:325-337), and butyric acid has been made by *Clostridium tyrobutyricum* (Wu and Yang (2003) Biotechnol. Bioeng. 82:93-102). Propionate and propanol have been made by fermentation from threonine by *Clostridium sp.* strain 17cr1 (Janssen (2004) Arch. Microbiol. 182:482-486). A yeast-like *Aureobasidium pullulans* has been used to make gluconic acid (Anantassiadis et al., (2005) Biotechnol. Bioeng. 91:494-501), by a mutant of *Aspergillis niger* (Singh et al., (2001) Indian J. Exp. Biol. 39:1136-43). 5-keto-D-gluconic acid was made by a mutant of *Gluconobacter oxydans* (Elfari et al., (2005) Appl Microbiol. Biotech. 66:668-674), itaconic acid was produced by mutants of *Aspergillus terreus* (Reddy and Singh (2002) Bioresour. Technol. 85:69-71), citric acid was produced by a mutant *Aspergillus niger* strain (Ikram-UI-Haq et al., (2005) Bioresour. Technol. 96:645-648), and xylitol was produced by *Candida guilliermondii* FTI 20037 (Mussatto and Roberto (2003) J. Appl. Microbiol. 95:331-337). 4-hydroxyvalerate-containing biopolyesters, also containing significant amounts of 3-hydroxybutyric acid 3-hydroxyvaleric acid, were produced by recombinant *Pseudomonas putida* and *Ralstonia eutropha* (Gorenflo et al., (2001) Biomacromolecules 2:45-57). L-2,3-butanediol was made by recombinant E. coli (Ui et al., (2004) Lett. Appl. Microbiol. 39:533-537).

Production of amino acids by fermentation has been accomplished using auxotrophic strains and amino acid analog-resistant strains of *Corynebacterium, Brevibacterium,* and *Serratia.* For example, production of histidine using a strain resistant to a histidine analog is described in Japanese Patent Publication No. 56008596 and using a recombinant strain is described in EP 136359. Production of tryptophan using a strain resistant to a tryptophan analog is described in Japanese Patent Publication Nos. 47004505 and 51019037. Production of isoleucine using a strain resistant to an isoleucine analog is described in Japanese Patent Publication Nos. 47038995, 51006237, 54032070. Production of phenylalanine using a strain resistant to a phenylalanine analog is described in Japanese Patent Publication No. 56010035. Production of tyrosine using a strain requiring phenylalanine for growth, resistant to tyrosine (Agr. Chem. Soc. Japan 50 (1) R79-R87 (1976), or a recombinant strain (EP263515, EP332234), and production of arginine using a strain resistant to an L-arginine analog (Agr. Biol. Chem. (1972) 36:1675-1684, Japanese Patent Publication Nos. 54037235 and 57150381) have been described. Phenylalanine was also produced by fermentation in *Eschericia coli* strains ATCC 31882, 31883, and 31884. Production of glutamic acid in a recombinant coryneform bacterium is described in US 6,962,805. Production of threonine by a mutant strain of E. coli is described in Okamoto and Ikeda (2000) J. Biosci Bioeng. 89:87-79. Methionine was produced by a mutant strain of *Corynebacterium lilium* (Kumar et al, (2005) Bioresour. Technol. 96: 287-294).

Useful peptides, enzymes, and other proteins have also been made by biocatalysts (for example, in US 6,861,237, US 6,777,207, US 6,228,630).

Target compounds produced in fermentation by biocatalysts may be recovered using various methods known in the art. Products may be separated from other fermentation components by centrifugation, filtration, microfiltration, and nanofiltration. Products may be extracted by ion exchange, solvent extraction, or electrodialysis. Flocculating agents may be used to aid in product separation. As a specific example, bioproduced 1-butanol may be isolated from the fermentation medium using methods known in the art for ABE fermentations (see for example, Durre, Appl. Microbiol. Biotechnol. 49:639-648 (1998), Groot et al., Process. Biochem. 27:61-75 (1992), and references therein). For example, solids may be removed from the fermentation medium by centrifugation, filtration, decantation, or the like. Then, the 1-butanol may be isolated from the fermentation medium using methods such as distillation, azeotropic distillation, liquid-liquid extraction, adsorption, gas stripping, membrane evaporation, or pervaporation. Purification of 1,3-propanediol from fermentation media may be accomplished, for example, by subjecting the reaction mixture to extraction with an organic solvent, distillation, and column chromatography (US 5,356,812). A particularly good organic solvent for this process is cyclohexane (US 5,008,473). Amino acids may be collected from fermentation medium by methods such as ion-exchange resin adsorption and/or crystallization. Alcohols are typically recovered using distillations and molecular sieves.

### Fermentation

Any biocatalyst, such as those described above, is used for fermentation of fermentable sugars produced by the present process. Fermentation conditions used with a particular biocatalyst may be as described in the above cited references, or as known to one skilled in the art.

As an example, the following describes a large-scale fermentation using *Zymomonas mobilis* for production of ethanol. The desired *Z. mobilis* cells are grown in shake flasks in semi-complex medium at about 30 °C to about 37 °C with shaking at about 150 rpm in orbital shakers and then transferred to a 10 L seed fermenter containing similar medium. The seed culture is grown in the seed fermenter anaerobically until OD₆₀₀ is between 3 and 6, when it is transferred to the production fermenter where the fermentation parameters are optimized for ethanol production. Typical inoculum volumes transferred from the seed tank to the production tank range from about 2% to about 20% v/v. The fermentation medium may be composed solely of hydrolysate, or may include components additional to the hydrolysate such sorbitol or mannitol at a final concentration of about 5 mM as described in US 7,629,156. The fermentation may be a batch process, fed-batch process, or continuous process, Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA, or Deshpande, Mukund V., Appl. Biochem. Biotechnol., 36, 227, (1992).

The fermentation is typically controlled at pH 4.5 - 6.5 using caustic solution (such as ammonium hydroxide, potassium hydroxide, or sodium hydroxide) and either sulfuric or phosphoric acid. The temperature of the fermentor is controlled at 30 °C - 37 °C. In order to minimize foaming, antifoam agents (any class- silicone based, organic based, etc.) may be added to the vessel as needed.

Any set of conditions described above, and additional variations in these conditions that are well known in the art, are suitable conditions for production of ethanol by xylose-utilizing recombinant *Zymomonas* cells. Typically cultures are incubated without supplemented air, oxygen, or other gases (which may include conditions such as anaerobic, microaerobic, or microaerophilic fermentation), for at least about 20 hours, and may be run for about 48 hours, 120 hours or longer.

In addition, fermentation may be performed using a simultaneous saccharification and fermentation (SSF) process or hybrid saccharification and fermentation (HSF) process. In HSF partial saccharification is carried out prior to addition of *Zymomonas* cells, then further saccharification and fermentation occur simultaneously. The second stage simultaneous saccharification and fermentation may be performed as described in US Patent Application Publication 2011/0318803, issued as US 8,647,850. In this process *Zymomonas* cells are grown under conditions of low impeller agitation with high concentration of insoluble solids in a saccharification-fermentation mixture during a simultaneous saccharification and fermentation reaction for the production of high concentrations of ethanol.

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the scope thereof, can make various changes and modifications of the invention to adapt it to various uses and conditions.

The meaning of abbreviations is as follows: "hr" means hour(s), "min" means minute(s), "sec" means second(s), "d" means day(s), "L" means liter(s), "mL" means milliliter(s), "µL" means microliter(s), "g" means grams, "µg" means microgram(s), "ng" means nanogram(s), "g/L" means grams per liter, "mM" means millimolar, "µM" means micromolar, "nm" means nanometer(s), "µmol" means micromole(s), "pmol" means picomole(s), "OD₆₀₀" means optical density measured at 600 nm, "w/v" means weight per volume, "w/w" means weight per weight, "sacc" is saccharification, "ferm" is fermentation, "av" is average, "gluc" is glucose, "xyl" is xylose, "arab" is arabinose.

### General Methods

### Saccharification

Pretreated corn stover was enzymatically hydrolyzed using the enzyme mixture Accellerase ® Trio™ (Genencor, Palo Alto, CA). The solids loading for the hydrolysis was kept at 20% or 25% by weight. Prior to saccharification, the amount of sulfuric acid needed for pH adjustment to 5.3 was determined by titration. The saccharification was carried out for 48 h at pH 5.3, 47 °C, and 250 rpm agitation. Each hydrolysis was conducted in a 1.0 L baffled flask with 250 g total saccharification mixture. Samples were taken at 24 hr and 48 hr for HPLC analysis.

### Biocatalyst

### Strain CD2

*Zymomonas mobilis* strain C2D was used as the biocatalyst in fermentation producing ethanol in Examples 1-3. Strain C2D was derived from strain AR3 7-31, which was derived from strain ZW705, which is disclosed in US 8,247,208. Strain AR3 7-31 was isolated following growth of strain ZW705 in a trubidostat as described in US 2012/0329114; the strain is also called therein Adapted 7-31. In this continuous flow culture device the concentration of ammonium acetate and ethanol was increased over time in a hydrolysate medium. The entire genome of AR3 7-31 was sequenced and compared to the sequence of the ZW705 genome. Strain AR3 7-31 was found to have a genetic modification in the zmo1432 open reading frame of the *Zymomonas mobilis* genome (NCBI Reference: NC_006526.2), in which zmo1432 is annotated as encoding a "fusaric acid resistance protein". The effect of this modification is to improve the behavior of the strain in a hydrolysate medium.

Further modifications were made to strain AR3 7-31 to improve its performance. An arabinose utilization pathway was added by introducing araB, araA, and araD gene expression as a Pgap-BAD operon as described in US 2011/0143408. The Pgap-BAD operon was integrated into the pnp locus, causing expression of a C-terminal truncated protein from the pnp locus, as disclosed in US 2013/0157331. An araE arabinose-proton symporter was expressed to improve arabinose utilization as disclosed in US 2011/0143408. Increased expression of ribose-5-phosphate isomerase activity was engineered by introducing an additional gene encoding RPI as disclosed in US 2012/0156746. The resulting modified C2D strain utilizes xylose and arabinose for production of ethanol in hydrolysate medium.

### Strain R70B1

*Zymomonas mobilis* strain ZW1-XA111 was prepared from strain ZW1 (ATCC 31821). ZW1 was engineered to express the four xylose utilization pathway genes: xylA, xylB, tkt, and tal as described above for ZW705. The xylA coding region was from *Actinoploanes missouriensis* (disclosed in US 2011/0318801) and variant high activity *Z. mobilis* glyceraldehyde-3-phosphate dehydrogenase gene promoters (disclosed in US 7,989,206) were used to express xylA and a tal/tkt operon. Integration of the xylA and xylB genes inactivated the gfor locus (US 7,741,119). The strain was engineered for increased expression of ribose-5-phosphate isomerase (Rpi) as disclosed in US Patent Application US-2012-0156746, and ribulose-phosphate 3-epimerase (Rpe) as disclosed in US Patent Application US-2013-0157331. The resulting strain was passaged for 4 doublings in xylose medium for adaptation, as described in US 7,629,156, during which a genetic modification occurred in the zmo0976 open reading frame of the *Zymomonas mobilis* genome (NCBI Reference: NC_006526.2), which codes for an enzyme that has NADPH-dependent xylose reductase activity that is able to convert xylose to xylitol (Agrawal and Chen (2011) Biotechnol Lett.; online publication 7/01/2011). Disruption of zmo0976 reduces NADPH-dependent xylose reductase activity by greater than 90%, as disclosed in US Patent Application US-2013-0157332, and improves growth on xylose-containing medium.

The strain was engineered to express the *E. coli* araBAD operon which encodes L-ribulose kinase, L-arabinose isomerase, and L-ribulose-5-phosphate-4-epimerase, respectively, which provide an arabinose assimilation pathway, in conjunction with transketolase and transaldolase activities that were introduced for xylose utilization (US 5,843,760). Integration of an araBAD operon inactivated the pnp gene encoding polynucleotide phosphorylase thereby providing improving xylose utilization and ethanol production as disclosed in US Patent Application US-2013-0157331. The resulting strain was passaged for 10 doublings in xylose medium for adaptation, as described in U.S 7,629,156.

To further improve xylose utilization the strain was engineered to express a heterologous arabinose-proton symporter, encoded by the araE gene of *E. coli* as disclosed in US 2011/143408. The chloramphenicol resistance marked used in this step was removed from the genome producing the ZW1-XA111 strain.

*Z. mobilis* strain R70B1 was developed from ZW1-XA111 through serial adaptation. Briefly, ZW1-XA111 was revived and placed into corn stover hydrolysate Y018

After all of the glucose and approximately half of the xylose was consumed, 10 vol% of the culture was removed and placed into a new tube of corn stover hydrolysate. This was repeated for 70 transfers. After the 70th transfer, the population was plated to isolate single colonies, with R70B1 being one of the isolated colonies.

### Stover hydrolysate Y018

### Pretreatment

Corn stover was pretreated prior to enzymatic hydrolysis using low ammonia methods described in US 7,932,063. A horizontal Eirich 340 L reactor vessel containing a jacket for passing steam around the body of the vessel was used for pretreatment to generate pretreated cob named Y018. The vessel was loaded with stover to reach 60 v% (40.8 kg). The stover was reduced to less than 1/32 inch (0.8 mm).

The stover had a wet loose bulk density of 0.200 g/cm³ and 8.0 wt% moisture. After stover was charged, vacuum was applied to the vessel to reach -0.9 barg (-90 kPag) prior to introduction of ammonium hydroxide solution to give 8 wt% NH₃ relative to dry weight biomass and 65 wt% solids inside the vessel. In all batches, the reactor agitator was set to 40 Hz (42 rpm) and steam pressure on the jacket was 3.2 barg. Once the ammonia solution was added, steam at 16 barg (1600 kPag) was added to the vessel to raise and maintain the internal vessel temperature to 140 °C. This temperature was held for 30 minutes. At the end of pretreatment, the reactor was depressurized through a vent condenser to reach atmospheric pressure. Vacuum was subsequently applied to reach - 0.9 barg (-90 kPag) to lower the temperature and to remove additional ammonia and water from the pretreated stover prior to opening the bottom valve of the vessel and recovering the pretreated biomass. Final wt% of solids for pretreated stover batches Y018-X, Y018-5, and Y018-10 prepared as described was 55.5%, 67.0%, and 64.4%, respectively.

### Saccharification

The Y018 hydrolysate was generated in a 1000 L fermenter using a mixture of the pretreated corn stover from batches described above, treated with Accellerase® TRIO. A water heel was added to the fermenter and sterilized with jacket heat to 121 °C, and held for 20 minutes. The water was cooled to 47 °C and the pretreated stover mixture was added through a port on the top of the tank; the slurry was approximately 8 wt% solids at this time. The pH was adjusted to 5.3 with 5 wt% H₂SO₄ and the enzyme was added. The enzyme dosage was the equivalent to 14 mg of protein per g of glucan+xylan in the total stover to be added to the reactor. Over the following 24 hours, the remaining stover was added to a target of 25 wt% solids, with the pH controlled to 5.3 with 5 wt% H₂SO₄. The fermenter was controlled at 47 °C and pH 5.3 for approximately 72 hours. At the end of this time period, 20 liters was drawn off for use in these experiments, and the remaining contents of the vessel were fermented. A sample of the hydrolysate was analyzed and the remainder was stored refrigerated until use. The results of the sample analysis are shown in Table 3.

**Table 3: End of saccharification hydrolysate properties for Y018**

| | |
|---|---|
| Monomer Glucose (g/L) | 63.45 |
| Oligomer Glucose (g/L) | 18.68 |
| Monomer Xylose (g/L) | 38.81 |
| Oligomer Xylose (g/L) | 16.16 |
| Monomer Arabinose (g/L) | 8.02 |
| Oligomer Arabinose (g/L) | 3.38 |
| Lactic Acid (g/L) | 0.41 |
| Solids content (wt%) | 25.0% |

### Seed Culture Preparation

To prepare a seed culture, Z. *mobilis* cells of strain C2D were grown in medium containing 10 g/L yeast extract, 2 g/L KH₂PO₄ , 5 g/L MgSO₄.7H₂O and 150 g/L glucose at 33°C. The *Z. mobilis* seed culture was grown overnight at 33 °C, with 150 rpm agitation, under a largely anaerobic conditions. The seed was harvested when it reached late log phase of growth.

### Fermentation

The hydrolysate generated from saccharification (as described above) was used for fermentation. Fermentations were conducted at a working volume of 275 ml in a 1 L unbaffled flask at 33 °C, initial pH 5.8, 120 rpm agitation for 48 hr. The seed culture described above was inoculated into the hydrolysate to initiate fermentation. The inoculum ratios were at 2% w/v or 10% w/v depending on the design of the experiments. The flasks were capped with rubber stoppers pierced with a needle to vent carbon dioxide formed during fermentation. Samples were taken during the course of fermentation. Glucose, xylose, arabinose, acetic acid, acetamide and ethanol profiles were measured using HPLC analysis.

### HPLC Analysis

The concentrations of glucose, xylose, arabinose, and ethanol in fermentation cultures were analyzed using high-performance liquid chromatography (HPLC) with a Biorad Aminex HPX-87 H column (Hercules, CA). The column temperature was maintained at 60 °C and the mobile phase (10mM H₂SO₄) was kept at 0.6 mL/min flow rate.

### HPLC Analysis-2

The concentrations of monomer glucose, xylose, and arabinose generated in saccharification and fermentation were analyzed using HPLC on a Biorad Aminex HPX-P column with an ionic form H+/CO3 deashing guard column and an in-line prefilter. The column temperature was held at 80-85°C and the mobile phase (0.2 µm filtered DI water) was kept at a 0.6 mL/min flow rate.

The concentration of ethanol generated in fermentation was analyzed using HPLC on a Biorad Aminex HPX-H column with a Carbo-H guard column and an in-line prefilter. The column temperature was held at 55-65°C and the mobile phase (0.01N sulfuric acid made with 0.2 µm filtered DI water) was kept at 0.6 mL/min flow rate.

### Example 1

### Comparison between pretreatments at different percent solids using constant-profile pretreatment

Corn stover was milled and passed through a 1 mm screen. The milled stover (52.5 g of dry weight equivalent) was mixed with ammonium hydroxide solution and distilled water to achieve 8 w/w % NH₃ loading and 40.0% solids. The mixture was then charged into a 600 mL stainless steel Parr reactor (Parr Instrument, Moline, IL) and bolted shut. The Parr reactor was a non-stirred reactor. The reactor was heated to 140 °C in a fluidized sand bath for 40 min. After the hold time, the reactor was cooled to less than 50 °C in an ice bath. The contents were then removed from the reactor and dried in a vacuum dryer overnight at 65 °C. These steps were separately repeated, except using 52.5% solids and 65.0% solids. The resulting dried pretreated corn stover samples were then saccharified and fermented as described in General Methods. Samples were taken at different times during saccharification and fermentation, and were analyzed for glucose, xylose, arabinose, and ethanol as described in General Methods.

The results are given in Figures 1, 2, and 3 for 40 %solids, 52.5% solids, and 65% solids experiments, respectively, with 24 hr and 48 hr being times of saccharification, and the 0, 26 and second 48 hr times referring to times of fermentation. The results showed that pretreatment at the lowest percent solids tested (40%) produced the highest ethanol yield compared to pretreatments with 52.5% and 65% solids.

### Example 2

### Comparison between gradient and non-gradient pretreatments

Three experiments were performed using a gradient of biomass solids (Experiment 1), 65% constant biomass solids (Experiment 2), and 40% constant biomass solids (Experiment 3). Each experiment had 3 stages of pretreatment, as outlined in Table 1.

**Table 1 Experimental design for pretreatments**

| | % Solids | | | |
|---|---|---|---|---|
| | Experiment 1 | Experiment 2 | Experiment 3 | Time (min) |
| 1^{st} Stage | 65 | 65 | 40 | 15 |
| 2^{nd} Stage | 52.5 | 65 | 40 | 15 |
| 3^{rd} Stage | 40 | 65 | 40 | 15 |

For Experiment 1, 55.3 g of 1 mm sized milled corn stover was mixed with 14.48 g of 29% ammonium hydroxide and 10.94 g of distilled water to create a stover mixture containing 14.86 w/w % of ammonia concentration at 65 w/w % solids. The mixture was then charged into a 600 mL stainless steel Parr reactor (non-stirred reactor) that was bolted shut. The reactor was heated to 140 °C in a fluidized sand bath for the 15 minute 1^{st} stage hold time as indicated in Table 1. After the hold time, the reactor was cooled to less than 50 °C in an ice bath. The contents were then removed from the reactor and dried in a vacuum dryer overnight at 65 °C.

For stage 2 of Experiment 1, the dried contents from first stage pretreatment were mixed with 14.48 g of 29% ammonium hydroxide and 30.19 g distilled water to achieve a mixture of 52.5% solids. The mixture was then pretreated in the Parr reactor for a 15 min hold time at 140 °C, heated in the sand bath. The reactor was then cooled to less than 50 °C in an ice bath. The contents were removed from the reactor and dried completely in a vacuum dryer overnight at 65 °C.

For stage 3 of Experiment 1, the dried materials from the second stage were mixed with 14.48 g of 29% ammonium hydroxide and 61.43 g of distilled water to achieve a mixture of 40% solids. The mixture was then pretreated in the Parr reactor for 15 min hold time at 140 °C, heated in the sand bath. After the hold time, the reactor was cooled to less than 50 °C in an ice bath. The contents were removed from the reactor and dried completely in a vacuum dryer overnight at 65 °C.

For experiments 2 and 3, the procedure was identical except that the % solids in all three stages in each experiment was kept constant at 65% and 40% solids, respectively. Table 2 gives the components of the pretreatment mixtures in all 3 experiments.

**Table 2 Recipes for pretreatment mixtures at different percent solids**

| | | | |
|---|---|---|---|
| %Solids | 65.0% | 52.5% | 40.0% |
| NH₃/(NH₃+H₂O) | 14.86% | 8.84% | 5.33% |
| Dry Stover (g) | 52.49 | 52.49 | 52.49 |
| Stover % Solids | 95 | 95 | 95 |
| As is Stover (g) | 55.33 | 55.31 | 55.31 |
| 29% NH₃ (g) | 14.48 | 14.48 | 14.48 |
| Water (g) | 10.94 | 30.19 | 61.43 |

The percent solids and ammonia concentration are graphed for the gradient Experiment 1, and the constant 40% solids Experiment 3 in Figure 4A and B, respectively. In the gradient experiment the ammonia concentration decreased due to the increased water added to reduce the % solids. The amount of ammonia relative to dry weight of biomass remained constant during the gradient experiment.

In all 3 experiments, after being pretreated in 3 stages, the stover was saccharified and fermented using the procedure described in General Methods. Samples were taken at different times during saccharification and fermentation, and were analyzed for glucose, xylose, arabinose, and ethanol as described in General Methods. Thus a comparison of gradient % solids in pretreatment was made with constant % solids in pretreatment, at 2 different concentrations.

The results are given in Figures 5, 6, and 7 for gradient of %solids, 65% solids, and 40% solids experiments, respectively, with 24 hr and 48 hr being times of saccharification, and the 0, 26 and second 48 hr times referring to times of fermentation. The results showed that the gradient pretreatment produced the highest ethanol yield as compared to the constant pretreatments.

### Example 3

### Comparison between gradient and non-gradient materials using direct steam injection or water addition with dry heat to provide the gradient

629.3 grams of corn stover, hammermilled through a 1 mm screen, were loaded into a 6.7 L agitated and jacketed horizontal reactor and aqueous ammonia and water were added to achieve the desired initial percent solids as listed for each experiment in Table 3.

For experiments 1, 2, and 3, steam was applied to the reactor jacket to raise and maintain the temperature at 140 °C with no further additions of materials into the reactor. The reaction mass was held at temperature for 40 minutes before being vented to atmospheric pressure and application of vacuum to remove excess ammonia.

For experiments 4 and 5, aqueous ammonia and water were added into the reactor to achieve 65 wt% solids initially and then water or steam (see Table 3) were added continuously over approximately 35 minutes to achieve 40 wt% solids. Steam addition to the jacket was again used to maintain temperature at 140 °C in both experiments. At the end of a total 40 minute hold time at temperature, the reactor was vented to atmospheric pressure and vacuum was applied to remove excess ammonia.

**Table 3 Ammonia, percent solids, heat, and moisture parameters for experiments 1-5**

| | Exp. 1 | Exp. 2 | Exp. 3 | Exp. 4 | Exp. 5 |
|---|---|---|---|---|---|
| Ammonia Loading | 0.08 g NH₃/g dry stover | | | | |
| Percent Solids | Constant at 40% | Constant at 52.5% | Constant at 65% | Gradient 65% to 40% | Gradient 65% to 40% |
| Heat Source | Dry Heat | Dry Heat | Dry Heat | Wet Heat (Steam Injection) | Dry Heat (Gradient achieved with water add ition) |
| Hold Time (min) | 40 | 40 | 40 | 40 | 40 |

The resulting pretreated biomass from each experiment was saccharified for 48 hr as described in General Methods. The sugar yields in the resulting hydrolysates were assessed by assaying for arabinose, xylose, and glucose by HPLC. The results in Figure 8 show that the glucose and total sugar yields from the gradient pretreatment experiments were greater than from the constant pretreatment experiments. The total sugar yields were at least about 4% greater for the gradient pretreatment samples than for the constant pretreatment samples.

The resulting hydrolysates from the experiments were fermented as described in General Methods, using either 2% v/v inoculum or 10% v/v inoculum. The results of the 2% inoculum experiment, with fermentation for 120 hr are graphed in Figure 9, and show that fermentation was more effective using hydrolysate from the gradient pretreatment than from the constant pretreatment experiments, as measured by glucose and xylose utilization, and ethanol production.

Results from the 10% inoculum experiment are shown in Figure 11. Figure 10 shows the initial sugars and ethanol concentrations, and Figure 11 shows the sugars and ethanol concentrations after 48 hr of fermentation. Ethanol yields were highest from fermentation of the hydrolysates from the gradient pretreatments as compared to hydrolysates from the constant profile pretreatments.

### Example 4 (Prophetic)

### Adjustment of the Gradient Profile through Manipulating Steam Quality

Corn stover, hammermilled through a 1 mm screen, is loaded into a reactor having a steam injection system, and ammonia solution and water are added to achieve the desired initial percent solids. The reactor is shut. Steam is added directly into the stover/ammonia solution/water mixture to increase the temperature to 120 - 200 °C. The temperature of the incoming steam is adjusted to achieve different extents of gradients at a given temperature range. The incoming steam pressures are between 3 and 16 bar gauge. By manipulating the incoming steam temperature, the amount of steam needed to maintain a given temperature can be manipulated. Therefore, various gradient profiles are engineered by manipulating the incoming steam.

### Example 5

### Comparison of 2-stage pretreatments at different 2^{nd} stage percent solids, temperature, and pressure

Corn stover was milled and passed through a 1/8" screen. The milled stover (483 kg dry weight equivalent) and ammonium hydroxide solution (14 wt%, 313 kg) were charged into an agitated and heat-traced 4800 L reactor. Anhydrous ammonia (14.3 kg) was injected into the vessel to target a total 12 w/w% NH₃ loading and 55% solids. The reactor was heated with direct steam injection to 6 barg (600 kPa gauge) pressure and held for 30 minutes (1^{st} stage). The reactor was then heated with direct steam injection to between 8 and 12 barg (800 and 1200 kPa gauge) and held for 10 minutes (2^{nd} stage) before being vented to atmospheric pressure, followed by application of vacuum to remove excess ammonia.

Pretreatment details are listed in Table 4. Increase in 2^{nd} stage pressure by direct steam injection produced correspondingly higher temperatures and lower percent solids as given in Table 4.

**Table 4 Ammonia loading, pressure, temperature, and percent solids for experiments 1-5.**

| Exp # | NH₃% w/w dry mass | % Solids before Steam Injection | Av 1^{st} Stage Pressure (barg) | Av 1^{st} Stage Temp (°C) | % Solids After 1^{st} Stage | Av 2^{nd} Stage Pressure (barg) | Av 2^{nd} Stage Temp (°C) | % Solids at End of 2^{nd} Stage |
|---|---|---|---|---|---|---|---|---|
| 1 | 12.1 | 53.8 | 6.0 | 127.9 | 47.0 | 7.9 | 135.3 | 44.7 |
| 2 | 11.9 | 54.1 | 6.0 | 127.7 | 46.1 | 8.9 | 142.1 | 43.4 |
| 3 | 12.3 | 53.8 | 6.0 | 126.0 | 46.8 | 9.9 | 145.2 | 43.5 |
| 4 | 11.6 | 53.9 | 6.0 | 128.2 | 46.5 | 10.9 | 150.8 | 42.7 |
| 5 | 11.9 | 53.9 | 6.1 | 126.4 | 46.5 | 11.9 | 154.1 | 41.8 |

The resulting pretreated biomass from each experiment was saccharified for 48 hr using the procedure described in General Methods with solids loading at 25% by weight and the following modifications: each hydrolysis was conducted in a 125 ml flask with 47 g total saccharification mixtures at 200 rpm. The sugar yields in the resulting hydrolysates were assessed by assaying for arabinose, xylose and glucose by HPLC at the end of saccharification.

The resulting hydrolysates from the experiments were fermented as described in General Methods, using 10% v/v of strain R70B1 inoculum. R70B1 seed culture was prepared as in General Methods for the C2D strain except that the medium contained 1g/L MgSO₄.7H₂O. Samples were assayed by HPLC after 48 hr of saccharification and after 48 hr of fermentation as described in General Methods, HPLC Analysis-2.

Saccharification and fermentation results are provided in Table 5. 48 hr total sugars from saccharification and EtOH titers from fermentation increased monotonically from Experiment #1 to Experiment #5. These results demonstrate that biomass treatment at higher 2^{nd} stage pressures with correspondingly higher temperatures and lower percent solids produced more sugarsfrom saccharification and more EtOH in fermentation.

**Table 5 Titers at End of Saccharification and Fermentation**

| Exp # | 48 hr Sacc Gluc (g/L) | 48 hr Sacc Xyl (g/L) | 48 hr Sacc Arab (g/L) | 48 hr Sacc Gluc +Xyl +Arab (g/L) | 48 hr Ferm Gluc (g/L) | 48 hr Ferm Xyl (g/L) | 48 hr Ferm Arab (g/L) | 48 hr Ferm EtOH (g/L) |
|---|---|---|---|---|---|---|---|---|
| 1 | 68.7 | 42.0 | 4.0 | 114.7 | 0.4 | 1.3 | 0.0 | 62.2 |
| 2 | 67.3 | 43.1 | 5.3 | 115.7 | 0.4 | 1.3 | 0.0 | 62.6 |
| 3 | 66.9 | 43.1 | 5.7 | 115.7 | 0.4 | 1.5 | 0.0 | 64.1 |
| 4 | 66.9 | 43.4 | 5.6 | 115.9 | 0.4 | 1.6 | 0.0 | 64.5 |
| 5 | 68.3 | 44.8 | 5.6 | 118.7 | 0.4 | 1.9 | 0.0 | 64.8 |

### Example 6

### Comparison of 2-stage pretreatments at different initial percent solids

Corn stover was milled and passed through a 1/8" screen. The milled stover (484 kg dry weight equivalent) and varying amounts and strengths of ammonium hydroxide solution (239 - 508 kg and 18.2% to 8.6% NH₃) were charged into an agitated and heat-traced 4800 L reactor to target 9 w/w% NH₃. Anhydrous ammonia was injected into the vessel subsequently to achieve a total 12 w/w% NH₃ loading. Pre-steam solids loadings were varied between 44.3 and 57.3% (45 - 60% target). The reactor was heated with direct steam injection to 6 barg (600 kPa gauge) pressure and held for 30 minutes. The reactor was then heated with direct steam injection to 12 barg (1200 kPa gauge) and held for 10 minutes before being vented to atmospheric pressure, followed by application of vacuum to remove excess ammonia.

Pretreatment details are listed in Table 6. Pre-steam, 1^{st} stage, and 2^{nd} stage solids percent decrease monotonically from Experiment #1 to Experiment #4.

**Table 6 Ammonia loading, pressure, temperature, and percent solids for experiments 1-4.**

| Exp # | NH₃% (w/w dry mass) | % Solids Before Steaming | Average 1^{st} Stage Pressure (barg) | Average 1^{st} Stage Temp (°C) | % Solids After 1^{st} Stage | Average 2^{nd} Stage Pressure (barg) | Average 2^{nd} Stage Temp (°C) | % Solids at End of 2^{nd} Stage |
|---|---|---|---|---|---|---|---|---|
| 1 | 12.2 | 57.3 | 6.0 | 130.4 | 48.9 | 11.8 | 157.3 | 44.3 |
| 2 | 11.8 | 54.3 | 6.4 | 125.3 | 48.0 | 12.0 | 153.6 | 43.6 |
| 3 | 12.2 | 48.6 | 6.0 | 128.0 | 41.7 | 11.8 | 151.0 | 37.8 |
| 4 | 11.8 | 44.3 | 6.0 | 131.8 | 38.0 | 12.0 | 157.6 | 34.9 |

The resulting pretreated biomass from each experiment was saccharified for 48 hr using the procedure described in General Methods with solids loading at 25% by weight and the following modifications: each hydrolysis was conducted in a 125 ml flask with 47 g total saccharification mixture at 200 rpm. The sugar yields in the resulting hydrolysates were assessed by assaying for arabinose, xylose and glucose by HPLC at the end of saccharification.

The resulting hydrolysates from the experiments were fermented as described in General Methods, using 10% v/v R70B1 inoculum. R70B1 seed culture was prepared as in General Methods for the C2D strain except that the medium contained 1g/L MgSO₄.7H₂O. Samples were assayed by HPLC after 48 hr of saccharification and after 48 hr of fermentation as described in General Methods, HPLC Analysis-2. Results are given in Table 7.

Highest total sugar and EtOH titers were observed in Experiment #1 and lowest in Experiment #4. These results demonstrate that higher percent solids favor higher sugar and EtOH yields under the same nominal 2-stage pretreatment conditions.

**Table 7 Titers at End of Saccharification and Fermentation**

| Exp # | 48 hr Sacc Gluc (g/L) | 48 hr Sacc Xyl (g/L) | 48 hr Sacc Arab (g/L) | 48 hr Sacc Gluc +Xyl +Arab (g/L) | 48 hr Ferm Gluc (g/L) | 48 hr Ferm Xyl (g/L) | 48 hr Ferm Arabs (g/L) | 48 hr Ferm EtOH (g/L) |
|---|---|---|---|---|---|---|---|---|
| 1 | 70.5 | 45.5 | 5.6 | 121.6 | 0.4 | 2.5 | 0.0 | 65.6 |
| 2 | 70.1 | 43.8 | 5.4 | 119.3 | 0.4 | 1.8 | 0.0 | 63.3 |
| 3 | 62.7 | 41.6 | 5.3 | 109.6 | 0.4 | 2.1 | 0.0 | 63.6 |
| 4 | 62.5 | 41.5 | 5.3 | 109.3 | 0.4 | 2.5 | 0.0 | 61.9 |

## Claims

1. A process for producing fermentable sugars from cellulosic biomass comprising:
a) providing in a reaction vessel a reaction mixture comprising:
i) an initial solids concentration of cellulosic biomass of between 40% and 70%, measured as percent of dry biomass relative to the total mixture on a weight to weight basis; and
ii) ammonia, having a loading concentration of between 4% and 24% relative to dry weight of the biomass;
b) maintaining the reaction mixture between a temperature of 100 °C and 230 °C for a pretreatment reaction time having a starting time of t₀ and a final time of tₙ to produce a pretreated biomass, wherein at time t₀ the solids concentration of the reaction mixture is the initial solids concentration; and
c) contacting the pretreated biomass with a saccharification enzyme consortium under suitable conditions to produce fermentable sugars;
**characterised by** increasing the liquid content of the reaction mixture over the pretreatment reaction time of t₀ to tₙ in a gradient manner, whereby the solids concentration decreases by between 20% and 30% as compared to the initial solids concentration, to produce the pretreated biomass.

2. The process of claim 1 wherein the temperature of the reaction mixture is maintained at temperature between 100 °C and 200 °C for a reaction time.

3. The process of claim 2 wherein the reaction time is between 10 minutes and 2 hours.

4. The process of claim 1 wherein the liquid content of the reaction mixture is increased by the introduction to the reaction vessel of an agent selected from the group consisting of steam, water, ammonia solution, and combinations thereof.

5. The process of claim 1 wherein the decrease in percent of biomass solids from t₀ to tₙ is linear, is monotonic but not linear, or is step wise with the concentration of solids being held constant at least once between t₀ and tₙ for a period of time.

6. The process of claim 1 wherein the ammonia is selected from the group consisting of ammonia solution, anhydrous ammonia, ammonia vapor, and mixtures thereof.

7. The process of claim 1 wherein the ammonia comprises recycled ammonia.

8. The process of claim 1 wherein the temperature is varied between t₀ and tₙ.

9. The process of claim 8 wherein the temperature is increased or decreased between t₀ and tₙ.

10. The process of claim 1 wherein the cellulosic biomass comprises cellulose, hemicellulose and lignin.

11. The process of claim 10 wherein the cellulosic biomass is selected from the group consisting of corn stover, corn cob, corn grain fiber, grasses, beet pulp, wheat straw, wheat chaff, oat straw, barley straw, barley hulls, hay, rice straw, rice hulls, switchgrass, miscanthus, cord grass, reed canary grass, waste paper, sugar cane bagasse, sorghum bagasse, sorghum stover, soybean stover, components obtained from milling of grains, trees, branches, roots, leaves, wood chips, sawdust, palm waste, shrubs and bushes, vegetables, fruits, flowers and animal manure.

12. The process of claim 1 wherein the fermentable sugars provide a carbohydrate source for a biocatalyst for a fermentation process.

13. The process of claim 1 wherein the fermentable sugars are produced in at least 3% greater yield than yield from a process with constant biomass solids concentration.

14. The process of claim 1 wherein the fermentable sugars are contacted with a biocatalyst for the production of a target compound.

15. The process of Claim 14 wherein the target compound is selected from the group consisting of ethanol, methanol, propanol, butanol, 1, 3-propanediol, glycerol, xylitol, mannitol, lactic acid, acetic acid, formic acid, succinic acid, glutamic acid, 3-hydroxypropionic acid, fumaric acid, maleic acid and butyric acid.

## Patentansprüche

1. Verfahren zum Herstellen von gärungsfähigen Zuckern aus cellulosischer Biomasse, umfassend:
a) Bereitstellen einer Reaktionsmischung in einem Reaktionsgefäß, umfassend:
i) eine anfängliche Feststoffkonzentration cellulosischer Biomasse zwischen 40% und 70%, gemessen als Prozentsatz der trockenen Biomasse bezüglich der Gesamtmischung auf einer Basis von Gewicht-zu-Gewicht; und
ii) Ammoniak, das eine Ladekonzentration zwischen 4% und 24% bezüglich des Trockengewichts der Biomasse aufweist;
b) Aufrechterhalten der Reaktionsmischung bei einer Temperatur zwischen 100 °C und 230 °C für eine Vorbehandlungszeit, die eine Startzeit von t₀ und eine Endzeit von tₙ aufweist, um eine vorbehandelte Biomasse herzustellen, wobei am Zeitpunkt t₀ die Feststoffkonzentration der Reaktionsmischung die anfängliche Feststoffkonzentration ist; und
c) Kontaktieren der vorbehandelten Biomasse mit einem Verzuckerungsenzym-Konsortium unter geeigneten Bedingungen, um gärungsfähige Zucker herzustellen;
**gekennzeichnet durch** das Erhöhen des Flüssigkeitsgehalts der Reaktionsmischung über die Vorbehandlungs-Reaktionszeit von t₀ bis tₙ in der Art eines Gradienten, wobei sich die Feststoffkonzentration um zwischen 20% und 30% im Vergleich zu der anfänglichen Feststoffkonzentration reduziert, um eine vorbehandelte Biomasse herzustellen.

2. Verfahren nach Anspruch 1, wobei die Temperatur der Reaktionsmischung bei einer Temperatur zwischen 100 °C und 200 °C für eine Reaktionszeit aufrechterhalten wird.

3. Verfahren nach Anspruch 2, wobei die Reaktionszeit zwischen 10 Minuten und 2 Stunden beträgt.

4. Verfahren nach Anspruch 1, wobei der Flüssigkeitsgehalt der Reaktionsmischung durch das Einführen eines Mittels in das Reaktionsgefäß erhöht wird, das ausgewählt ist aus der Gruppe bestehend aus Dampf, Wasser, Ammoniaklösung und Kombinationen davon.

5. Verfahren nach Anspruch 1, wobei die Reduktion des Prozentsatzes der Biomassen-Feststoffe von t₀ bis tₙ linear ist, gleichbleibend, aber nicht linear ist, oder schrittweise ist, wobei die Feststoffkonzentration wenigstens einmal zwischen t₀ und tₙ für einen Zeitraum konstant gehalten wird.

6. Verfahren nach Anspruch 1, wobei das Ammoniak ausgewählt ist aus der Gruppe bestehend aus Ammoniaklösung, anhydrischem Ammoniak, Ammoniakdampf und Mischungen davon.

7. Verfahren nach Anspruch 1, wobei das Ammoniak wiederaufbereitetes Ammoniak umfasst.

8. Verfahren nach Anspruch 1, wobei die Temperatur zwischen t₀ und tₙ variiert wird.

9. Verfahren nach Anspruch 8, wobei die Temperatur zwischen t₀ und tₙ erhöht oder reduziert wird.

10. Verfahren nach Anspruch 1, wobei die cellulosische Biomasse Cellulose, Hemicellulose und Lignin umfasst.

11. Verfahren nach Anspruch 10, wobei die cellulosiche Biomasse ausgewählt ist aus der Gruppe bestehend aus Maisstengel, Maiskolben, Maiskornfaser, Gräsern, Rübenpulpe, Weizenstroh, Weizenspreu, Haferstroh, Gerstenstroh, Gerstenschalen, Heu, Reisstroh, Reisschalen, Rutenhirse, Miscanthusgras, Schlickgras, Rohrglanzgras, Altpapier, Zuckerrohrbagasse, Hirsenbagasse, Hirsestengel, Sojabohnenstengel, Komponenten, die von dem Mahlen von Getreide, Bäumen, Zweigen, Wurzeln, Blättern, Hackspänen, Sägemehl, Palmenabfall, Sträuchern und Büschen, Gemüse, Obst, Blumen und Tierdung erhalten werden.

12. Verfahren nach Anspruch 1, wobei die gärungsfähigen Zucker eine Kohlenhydratquelle für einen Biokatalysator für ein Gärungsverfahren bereitstellen.

13. Verfahren nach Anspruch 1, wobei die gärungsfähigen Zucker in einer Ausbeute hergestellt werden, die wenigstens 3% höher als die Ausbeute von einem Verfahren mit einer konstanten Biomassen-Feststoffkonzentration ist.

14. Verfahren nach Anspruch 1, wobei die gärungsfähigen Zucker für die Herstellung einer Zielverbindung mit einem Biokatalysator kontaktiert werden.

15. Verfahren nach Anspruch 14, wobei die Zielverbindung ausgewählt ist aus der Gruppe bestehend aus Ethanol, Methanol, Propanol, Butanol, 1,3-Propandiol, Glycerol, Xylitol, Mannitol, Milchsäure, Essigsäure, Ameisensäure, Bernsteinsäure, Glutaminsäure, 3-Hydroxypropionsäure, Fumarsäure, Maleinsäure und Buttersäure.

## Revendications

1. Procédé de production de sucres fermentables à partir d'une biomasse cellulosique comprenant :
a) la fourniture dans un récipient réactionnel d'un mélange réactionnel comprenant :
i) une concentration initiale en solides de biomasse cellulosique entre 40 % et 70 %, mesurée en pourcentage de biomasse sèche par rapport au mélange total sur une base poids/poids ; et
ii) de l'ammoniac, présentant une concentration de chargement entre 4 % et 24 % par rapport au poids sec de la biomasse ;
b) le maintien du mélange réactionnel entre une température de 100 °C et 230 °C pendant un temps réactionnel de prétraitement présentant un temps de début t₀ et un temps final tₙ pour produire une biomasse prétraitée, dans lequel au temps t₀, la concentration en solides du mélange réactionnel est la concentration initiale en solides ; et
c) la mise en contact de la biomasse prétraitée avec un groupe d'enzymes de saccharification dans des conditions appropriées pour produire des sucres fermentables ;
**caractérisé par** une augmentation de la teneur en liquide du mélange réactionnel pendant le temps réactionnel de prétraitement allant de t₀ à tₙ en gradient, selon laquelle la concentration en solides diminue entre 20% et 30% par rapport à la concentration initiale en solides, pour produire la biomasse prétraitée.

2. Procédé selon la revendication 1, dans lequel la température du mélange réactionnel est maintenue à une température entre 100 °C et 200 °C pendant un temps réactionnel.

3. Procédé selon la revendication 2, dans lequel le temps réactionnel se situe entre 10 minutes et 2 heures.

4. Procédé selon la revendication 1, dans lequel la teneur en liquide du mélange réactionnel est augmentée par l'introduction dans le récipient réactionnel d'un agent sélectionné dans le groupe consistant en la vapeur d'eau, l'eau, une solution ammoniacale, et les combinaisons de celles-ci.

5. Procédé selon la revendication 1, dans lequel la diminution en pourcentage des solides de la biomasse de t₀ à tₙ est linéaire, est monotone mais non linéaire, ou s'effectue par étape, la concentration en solides étant maintenue à un niveau constant au moins une fois entre t₀ et tₙ pendant un certain temps.

6. Procédé selon la revendication 1, dans lequel l'ammoniac est sélectionné dans le groupe consistant en une solution ammoniacale, l'ammoniac anhydre, la vapeur d'ammoniac, et les mélanges de ceux-ci.

7. Procédé selon la revendication 1, dans lequel l'ammoniac comprend de l'ammoniac recyclé.

8. Procédé selon la revendication 1, dans lequel la température varie entre t₀ et tₙ.

9. Procédé selon la revendication 8, dans lequel la température est augmentée ou diminuée entre t₀ et tₙ.

10. Procédé selon la revendication 1, dans lequel la biomasse cellulosique comprend une cellulose, une hémicellulose et une lignine.

11. Procédé selon la revendication 10, dans lequel la biomasse cellulosique est sélectionnée dans le groupe consistant en une canne de maïs, une rafle de maïs, les fibres de grain de maïs, les graminées, une pulpe de betterave, une paille de blé, une balle de blé, une paille d'avoine, une paille d'orge, les coques d'orge, un foin, une paille de riz, les balles de riz, le panic érigé, le miscanthus, les spartines, l'alpiste roseau, les vieux papiers, une bagasse de canne à sucre, une bagasse de sorgho, une paille de sorgho, une paille de soja, les composants obtenus à partir du broyage de grains, d'arbres, de branches, de racines, de feuilles, de copeaux de bois, de sciure, de résidu de palme, d'arbustes et de buissons, les légumes, les fruits, les fleurs et un fumier animal.

12. Procédé selon la revendication 1, dans lequel les sucres fermentables fournissent une source de glucides pour un biocatalyseur pour un processus de fermentation.

13. Procédé selon la revendication 1, dans lequel les sucres fermentables sont produits avec un rendement d'au moins 3 % supérieur par rapport à un rendement issu d'un procédé avec une concentration constante en solides de biomasse.

14. Procédé selon la revendication 1, dans lequel les sucres fermentables sont mis en contact avec un biocatalyseur pour la production d'un composé cible.

15. Procédé selon la revendication 14, dans lequel le composé cible est sélectionné dans le groupe consistant en l'éthanol, le méthanol, le propanol, le butanol, le 1,3-propanediol, le glycérol, le xylitol, le mannitol, l'acide lactique, l'acide acétique, l'acide formique, l'acide succinique, l'acide glutamique, l'acide 3-hydroxypropanoïque, l'acide fumarique, l'acide maléique et l'acide butyrique.
